# EUROPEAN PATENT SPECIFICATION

(11) **EP 1 988 909 B1**
(45) Date of publication and mention of the grant of the patent: **21.10.2009**
(21) Application number: 07801465.1
(22) Date of filing: 30.07.2007
(51) Int. Cl.: A61K 31/765, A61P 17/02

(54) **USE OF POLYETHYLENE GLYCOL IN INFLAMMATION RELATED TOPICAL DISORDERS OR DISEASES AND WOUND HEALING**
VERWENDUNG VON POLYETHYLENGLYCOL BEI ENTZÜNDUNGSBEDINGTEN TOPISCHEN ERKRANKUNGEN ODER ERKRANKUNGEN UND WUNDHEILUNG
UTILISATION DE POLYÉTHYLÈNE GLYCOL DANS LES TROUBLES OU MALADIES TOPIQUES LIÉS À L'INFLAMMATION ET DANS LA CICATRISATION DES PLAIES

(30) Priority: 28.07.2006 GB 0615016
(43) Date of publication of application: 12.11.2008
(73) Proprietor: Flen Pharma N.V., 2650 Edegem (BE)
(72) Inventor: VAN DEN PLAS, Dave, B-2100 Deurne (BE); DE SMET, Kris, B-2400 Mol (BE); SOLLIE, Philippe, B-2980 Zoersel (BE)
(74) Representative: Bird, William Edward
(86) International application number: PCT/EP2007/006714
(87) International publication number: WO 2008/012107

(56) References cited:
- WO-A-03/045293
- WO-A-20/06134279
- US-A1- 2003 129 209
- US-A1- 2005 192 357

## Description

### FIELD OF THE INVENTION

The present invention relates to compositions and their use as a cosmetic product, dietary product, medicament or as a medical device for the topical treatment of wounds or for reducing or helping to reduce inflammation, for enhancing the repair of damaged skin, mucosa and/or wounds and/or for the topical alleviation of inflammation related symptoms.

### BACKGROUND OF THE INVENTION

Polyethylene glycol (PEG) is a well-known component as a basis for pharmaceutical formulations or for modulating the viscosity of ointments and cosmetics. It is used as skin penetration enhancer and as a wetting agent for hydrophobic substances facilitating their dispersion. PEG has been used for the derivatization of therapeutic proteins to increase their solubility, stability and circulation time and/or decrease immunogenicity. PEG based polymers with tensioactive properties are used as emulsifiers, solubilisers and wetting agents in dermatological and cosmetic preparations, toiletries, perfumes as well as in industrial applications. PEG-linked polyamines have been found to increase transdermal delivery of topical therapeutic drugs.

PEG has also been demonstrated to have a neuroprotective effect, which is in part based on its ability to rescue neurons and their axons by repairing the plasma membranes.

The potential pharmaceutical activity of this compound in dermatological applications has been widely overlooked. In general, varying concentrations of PEG are used in dermatological formulations. While some of these formulations have been suggested for wound-healing (Chapvil et al. (1991) J. Surg. Res. 51, 245-252), PEG itself has not been considered as an active ingredient as the direct effect of PEG itself has not been recognized.

Antimicrobial compositions comprising PEG have been described (Ambrose et al. (1991) Antimicrobial agents and chemotherapy 35, 1799-1803), based on a pro-inflammatory activity of PEG. Concentrations of PEG used to obtain the alleged pro-inflammatory effect of PEG are above 15%.

Other compositions comprising anti-inflammatory active compounds and further comprising PEG have been described in the prior art. In these compositions, PEG is used as an excipient. US2003129209 discloses water based gel compositions comprising the H1 antihistaminic agent loratidine, 3% Macrogol (PEG Mr 400), 0,8 % Carbopol and water. No other humectants are present apart from PEG. The composition is used for imflammatory indications of the skin such as urticaria, allergic skin reactions, itching redness sunburns and insect bites.

US2005192357 discloses an aerosol aqueous solution comprising a prostaglandin D receptor selective antagonist and 5 % Macrogol 400. Embodiments of the application refer to the use of compositions wherein, apart from PEG, no other humectants are present. The compositions are used for the topical treatment of pruritic symptoms, namely itching and associated inflammation on the skin and the mucous membranes, such as scabies urticaria excema, xerosa and atopic dermatitis.

WO2006134279 discloses water based compositions comprising metronidazole in a solution gel or lotion, also comprising 5% PEG 400, for treating dermatological conditions such as rosacea.

Furthermore, PEG is known as a component in intermediate compositions for the manufacture of eg wound dressings. WO2003045293, for example, discloses a wound dressing made of cotton which is impregnated by an aqueous solution comprising alginate, a crosslinker, a catalyst, and a polyethyleneglycol such as PEG 600. This aqueous solution comprising preferably 1,2 % PEG.

Inflammation is the first response of the immune system to infection or irritation and may be referred to as the innate cascade. Inflammation is characterized by the following quintet: redness (rubor), heat (calor), swelling (tumor), pain (dolor) and dysfunction of the organs involved (*functio laesa*).

Inflammation has two main components, a cellular and an exudative component. The exudative component involves the movement of fluid, usually containing many important proteins such as fibrin and immunoglobulins (antibodies). Blood vessels are dilated upstream of an infection (causing redness and heat) and constricted downstream while capillary permeability to the affected tissue is increased, resulting in a net loss of blood plasma into the tissue - giving rise to edema or swelling. The swelling distends the tissues, compresses nerve endings, and thus causes pain. The cellular component involves the movement of white blood cells from blood vessels into the inflamed tissue. The white blood cells, or leukocytes, take on an important role in inflammation; they extravasate (filter out) from the capillaries into tissue, and act as phagocytes, picking up bacteria and cellular debris. They may also aid by walling off an infection and preventing its spread. When inflammation of the affected site persists, released cytokines IL-1 and TNF will activate endothelial cells to upregulate receptors VCAM-1, ICAM-1, E-selectin, and L-selectin for various immune cells. Receptor upregulation increases extravazation of neutrophils, monocytes, activated T-helper and T-cytotoxic, and memory T and B cells to the infected site.

Neutrophils are characteristic of inflammation in the early stages - they are the first cells to appear in an infected area, and any section of recently inflamed (within a couple of days or so) tissue viewed under a microscope will appear packed with them. They are easily identified by their multilobed nuclei and granular cytoplasm and perform many important functions, including phagocytosis and the release of extracellular chemical messengers. Neutrophils only live for a couple days in these interstitial areas, so if the inflammation persists for a longer duration then they are gradually replaced by longer lived monocytes.

Various leukocytes are involved in the initiation and maintenance of inflammation. These cells can be further stimulated to maintain inflammation through the action of adaptive cascade through lymphocytes: T cells, B cells, and antibodies. These inflammation cells are mast cells, which release histamine and prostaglandin in response to activation of stretch receptors and macrophages which release TNF-α, IL-1 in response to activation of toll-like receptors.

The outcome of inflammation in a particular circumstance will be determined by the tissue in which the injury has occurred, and the injurious agent that is causing it. There are four possible results to inflammation, i.e. resolution, connective tissue scarring, abscess formation and ongoing or chronic inflammation. Resolution, or the complete reconstitution of damaged tissue, does not usually occur in the body. More commonly, the inflammation entails connective tissue scarring. Some 24 hours after inflammation in a wound first occurs, the wound healing response will commence. This response involves the formation of connective tissue to bridge the gap caused by injury, and the process of angiogenesis, the formation of new blood vessels, to provide nutrients to the newly formed tissue. Often healing cannot occur completely and a scar will form; for example after laceration to the skin, a connective tissue scar results which does not contain any specialized structures such as hair or sweat glands. Where the inflammation is accompanied by infection with pyogenic bacteria, abscess formation can occur. Finally, if the injurious agent continues, chronic inflammation will ensue. This process, marked by inflammation lasting many days, months or even years, may lead to the formation of a chronic wound. Chronic inflammation is characterized by a dominating presence of macrophages in the injured tissue, which extravasate via the same methods discussed above (ICAM-1 VCAM-1). These cells are powerful defensive agents of the body, but the toxins they release (including reactive oxygen species) are injurious to the organism's own tissues as well as invading agents. This is why chronic inflammation is almost always accompanied by tissue destruction. An abscess, or a collection of pus, can also form in chronic inflammation.

Besides wounding, inflammation of the skin can also be caused by other agents acting as irritating factors. Common inflammatory skin diseases include atopic dermatitis, acne, poison ivy, rosacea, and hives. Atopic dermatitis is often referred to as "eczema," which is a general term for the many types of dermatitis. Atopic dermatitis is the most common of the many types of eczema including contact eczema (such as most forms of occupational dermatitis), allergic contact eczema, seborrheic eczema, nubular eczema, neurodermatitis, stasis dermatisis and dyshidrotic eczema. Atopic dermatitis affects males and females equally and accounts for 10 to 20 percent of all referrals to dermatologists. An estimated 10 percent of infants and young children experience symptoms of the disease. Roughly 60 percent of these infants continue to have one or more symptoms of atopic dermatitis into adulthood. This means that more than 15 million people in the United States have symptoms of the disease. Psoriasis vulgaris is a chronic inflammatory skin disease, the prevalence rate of which is 2-3% in Caucasian populations. Most cases of psoriasis vulgaris are sporadic. Sporadic cases are characterized by inflammation triggered by skin lesions showing hyperproliferation of epidermal cells, abnormal differentiation of keratinocytes (for example, keratinocyte hyperproliferation), infiltration of activated helper T cells and monocytes, and release of proinflammatory cytokines. Acne Vulgaris is an inflammatory disease of the skin, caused by changes in the pilosebaceous units (skin structures consisting of a hair follicle and its associated sebaceous gland). The condition is most common during adolescence, affecting more than 85% of teenagers, but frequently continues into adulthood.

There remains a need for compositions for application to the skin which can be used to alleviate symptoms of inflammation, wound-healing and/or promote wound-regeneration.

### SUMMARY OF THE INVENTION

One aspect of the invention relates to the use of one or more forms of Polyethylene Glycol (PEG) with a Mr below 1500 as the sole anti-inflammatory component for the manufacture of a medicament in the form of a hydrogel or an aqueous solution comprising said one or more forms of Polyethylene Glycol (PEG) at a concentration between 0.5 and 5 % (w/w), for the treatment of inflammation of the skin or mucosa.

In particular embodiments, the one or more forms of Polyethylene Glycol (PEG) are at a concentration between 0.5 and 3 % (w/w), or at a concentration between 1 and 2,5 % (w/w).

In other particular embodiments, the said medicament contains no humectant apart from PEG.

In yet other particular embodiments the one or more forms of PEG have a Mr between about 200 and 700.

In other particular embodiments the hydrogel is a polyacrylate hydrocolloid with a concentration between 0,05-20%.

The inflammation which is treated in accordance with the present invention may in a wound or in an inflammatory skin or in a mucosa disease.

The treatment of inflammation in accordance with the use of the present invention is performed to prevent scar formation or to repair damaged skin or mucosa.

Another aspect of the present invention relates to a hydrogel or aqueous solution comprising one or more forms of PEG with a Mr below 1500 in a concentration of between 0.5 and 5 % (w/w) as the sole anti-inflammatory component for reducing inflammation of the skin or mucosa.

In particular embodiments, the one or more forms of Polyethylene Glycol (PEG) are at a concentration between 0.5 and 3 % (w/w), or at a concentration between 1 and 2,5 % (w/w).

According to certain embodiments the reduction in inflammation of the skin or mucosa promotes the healing of a wound, more particularly a chronic wound, and/or prevents scar tissue formation and/or enhances skin repair, more particularly in an inflammatory skin disease.

According to particular embodiments, the hydrogel comprising composition or aqueous solution does not comprise a humectant apart from PEG.

According to other particular embodiments, the hydrogel is a polyacrylate hydrocolloid with a concentration between 0,05 and 20 %.

### FIGURE LEGENDS

- **Figure 1**: shows the toxicity of varying concentration of PEG400 on cultivated cells (A: keratinocytes; B: macrophages; C: fibroblasts) according to particular embodiments of the present invention.
- **Figure 2**: shows the influence of varying concentrations of PEG400 on metalloproteinase expression by cultivated cells according to particular embodiments of the present invention.
- **Figure 3**: shows the influence of varying concentrations of PEG on VEGF expression by cultivated cells according to particular embodiments of the present invention.
- **Figure 4**: shows the influence of PEG derivatives on protein expression by cultivated cells (A: VEGF expression by fibroblasts; B: ProMMP9 expression by macrophages; C: IL-6 expression by macrophages; D: TGF-beta1 by macrophages) according to particular embodiments of the present invention.
- **Figure 5**: shows the influence of PEG derivatives on protein expression by cultivated cells (PBMC) (A: TNF-alpha expression; B: TGF-beta1 expression) according to particular embodiments of the present invention.
- **Figure 6**: shows the influence of PEG400 in an alginate hydrogel formulation on protein secretion by cultivated cells (A: fibroblasts; B: macrophages) according to particular embodiments of the present invention.
- **Figure 7**: shows the influence of PEG400 in a polyacrylate hydrogel formulation on protein secretion by cultivated cells according to particular embodiments of the present invention.
- **Figure 8**: shows the influence of PEG in a carboxymethylcellulose hydrogel formulation on protein secretion by cultivated cells according to particular embodiments of the present invention.
- **Figure 9**: shows the influence of PEG400 on VEGF expression by endothelial cells (SVEC) and fibroblast cells (3T3) according to particular embodiments of the invention.
- **Figure 10**: shows the influence of PEG400 in combination with another humectant - propylene glycol - on VEGF expression by endothelial cells (SVEC).
- **Figure 11**: shows the influence of PEG400 and another humectant on collagen III expression by fibroblast cells (3T3).

### DETAILED DESCRIPTION OF THE INVENTION

### Definitions

The term **Polyethylene glycol (PEG, Macrogol)** is used herein to refer to a condensation polymer of ethylene oxide (Mr 62) and water with general formula HO-(CH₂-CH₂-O)ₙ-H. The low molecular weight members from n=2 to n=4 are diethylene glycol (Mr 106), triethylene glycol (Mr 150) and tetraethylene glycol (Mr 194) respectively, which are produced as pure compounds. Where appropriate, the abbreviation (PEG) is used in combination with a numeric suffix which indicates the average molecular weight of the PEG. Different forms of PEGs are identified according to molecular weight (low: 200-1500; high:>1500). Alternatively, PEGs can be divided according to their resulting viscosity, whereby PEG 200-700 are viscous liquids. PEG 1500 are semi-solids with a greasy touch. PEG >1500 are solids with a waxy or paraffin like appearance. The fluid and semi-solid PEG compositions are hygroscopic (humectants), while the wax-like compositions are much less hygroscopic (Cooper & Gunn's Dispensing for Pharmaceutical Students, 12th Edition, Edited by SJ Carter; Pitman medical Publishing Co Ltd.; Martindale, The extra Pharmacopoeia, 28th edition, Edited by J.E.F. Reynolds, Pharmaceutical Press, London). A form of PEG or a PEG species is a PEG or PEG derivative with a specified average molecular weight.

The term **"PEG derivative",** as used herein, relates to PEG which is modified by the addition of one or more straight chain or branched C1-C6 alkyl groups. A functionalised PEG or PEG derivative is a PEG or PEG derivative (further) substituted with one or more groups selected from the group consisting of acid (carbonic acid, sulphonic acid), aldehyde, CN, OH, OR, SH, SR, NH₂ or NHR, wherein R= C₁ to C₄ chain.

The term **"alginates",** as used herein relates to pharmaceutically acceptable alginates, such as alginic acid and cationic alginates such as calcium, sodium, potassium and ammonium alginates.

The term **"topical"** in the context of application methods and compositions of the present application refers to the application (or suitability for application) to the surface of a body part, more specifically application to the skin, nails or hair, or mucous membranes such as the ear, nose, vagina, rectum, throat or the eye (e.g. retina), including damaged portions thereof.

The term **"dermal"** as used herein in the context of application methods or compositions relates to application to the skin.

The term **"hydrogel"** is a general term relating to one or more natural or synthetic polymer, that are colloidally dispersed in water (Martin et al. in Physical Pharmacy, 2nd ed. (1960), Lea & Febiger-Philadelphia).

The term **"Inflammation"** as used herein generally refers to the local accumulation of fluid, plasma proteins, and white blood cells that is initiated by physical injury, infection, or a local immune response. This is also known as an inflammatory response. Acute inflammation is the term used to describe early and often transient episodes, while chronic inflammation occurs when the infection persists or during autoimmune response. Many different forms of inflammation are seen in different diseases. The cells that invade tissues undergoing inflammatory responses are often called inflammatory cells or an inflammatory infiltrate [ImmunoBiology, the immune system in health and disease, Janeway & Travers, 3rd edition (1997), Churchill- Livingstone/Current Biology Limited/Garland Publishing Inc). Inflammation is typically associated with the production of pro-inflammatory cytokines such as TNFα, IL-1, MMPs etc.

The term **'inflammatory skin disease'** as used herein refers to an inflammatory reaction in the skin to an irritant often characterized internally by local accumulation of fluid, plasma proteins, and white blood cells and externally by redness of the skin, thickness and heat production. Inflammatory skin disorders include eczema, acne, rosacea and many others including psoriasis, hives, contact dermatitis (such as occupational dermatitis) and poison ivy.

The term **"wound healing"** as used herein refers to the reduction in size and/or severity of superficial wounds (injury only to stratum corneum and/or epidermis; e.g. sunburn), both partial thickness (injury only to the epidermis and superficial dermis, with no damage to the blood vessels) and full thickness (loss of dermis, dermal blood vessels affected) wounds, as well as internal wounds.

A **"solution"** as used herein refers a mixture of two or more components which form a homogenous molecular dispersion, i.e. a one-phase system (Martin et al. in Physical Pharmacy, 2nd ed., page 144 Lea & Febiger-Philadelphia).

The term **"humectant"** as used herein refers to a hygroscopic substance. Examples hereof are glycerol, propylene glycol and glyceryl triacetate. Other type of humectants are polyols like sorbitol, xylitol and maltitol, and polymeric polyols like polydextrose, and natural extracts like quillaia, lactic acid and urea. It includes PEG, which as a result of its hygroscopic properties is considered a humectant.

Compositions are disclosed that comprise low concentrations of one or more forms of PEG thereof having, as sole anti-inflammatory component, a therapeutic and/or beneficial effect, more particularly for the reduction and/or treatment of inflammation. More particularly, the compositions are provided for topical administration, whereby the presence of PEG, as sole anti-inflammatory component, and at low concentrations reduces and/or furthers the reduction of inflammation and/or promotes skin repair and prevents and/or reduces scar formation, and/or increases wound healing and/or promotes wound-repair. In particular embodiments, the compositions are provided for the reduction of inflammation or for furthering the reduction of inflammation and/or for the promotion of skin repair and for preventing and/or reducing scar formation and/or for the treatment of wounds and/or for the promotion of wound-repair, wherein PEG is the only pharmaceutically active ingredient in the composition having a direct anti-inflammatory activity.

The disclosed compositions are **characterized in that** the low concentrations of PEG present ensure an anti-inflammatory effect on the skin or mucosa, when present as sole anti-inflammatory compounds in these compositions. Such compositions may further comprise other active ingredients such as sunscreens (e.g. benzoic acid based UV absorbing compounds, anthranilic acid based UV absorbing compounds, salicylic acid based UV absorbing compounds, cinnamic acid based UV absorbing compounds, benzophenone based UV absorbing compounds). In some embodiments, the composition being used can contain warming agents (e.g. capsaicin or menthol) or antimicrobial ingredients (antibiotic, antifungal or antiviral agents). In the compositions disclosed herein, PEG is the sole anti-inflammatory ingredient (such compositions may further comprise growth-inhibiting antimicrobial agents, etc.). In further alternative embodiments, PEG is the only active ingredient present in the composition, more particularly does not comprise warming agents and/or other active ingredients.

The therapeutic effect of PEG in the (topical) treatment of inflammation or wound healing, more particularly when used in low concentrations, has not yet been reported. Thus, the present invention relates to the use of compositions comprising low concentrations of PEG, as sole anti-inflammatory components, in applications for which they had not previously been envisaged, more particularly for the reduction or assistance in reduction of inflammation.and/or to promote skin repair and reduce scar formation, and/or for treatment of wounds, e.g. in wound healing. Compositions used in the present invention comprising PEG as sole anti-inflammatory ingredient for the topical treatment of inflammation and/or wound healing, may further optionally contain active ingredients not directly affecting inflammation or wound healing. Additionally or alternatively, the compositions can further contain additives such as buffers, salts, preservatives, perfumes, lubricants and other compounds used in the manufacture of cosmetics and ointments. It is demonstrated herein that compositions comprising PEG and no other anti-inflammatory compounds can be used to reduce inflammation and to promote wound healing. Accordingly, in particular embodiments compositions are used which, besides PEG comprise only compounds or additives which have no anti-inflammatory or wound healing effects (or which are present in concentrations which are too low (or too high) to have an anti-inflammatory or wound healing effect) for use in the treatment of inflammatory skin and/or mucosal diseases and wounds.

According to particular embodiments, the one or more forms of PEG or PEG species which are used in the present invention have a molecular weight (Mr) ranging from 200 to 1500, such as PEG 200, 300, 400, 600, 800, 1000 and 1500. Low Mw PEGs are envisaged in view of their physicochemical characteristics, which allow easy manipulation. In particular embodiments one or more forms of PEG with a molecular weight within the 200 to 700 Mw range are used.

The PEG in compositions used in the present invention can be homogeneous (i.e. comprising only one form of PEG) or can be a mixture of varying ratio's of one or more forms of PEGs. Typically, according to the use of present invention, the total concentration of all different forms of PEG present in the composition is between 0,5 to 5 % (w/w).

The concentrations of PEG envisaged for use in the present invention range from about 1-5 % (w/w), particularly between 1-3% (w/w). The (total) PEG concentration is herein can be 1, 2, 3, 4 or 5 % (w/w). More particularly, the concentration of PEG envisaged in hydrogels used in the invention is lower than the concentration of PEG used in PEG-hydrogels, where PEG acts as wetting agent.

The PEG(s) used in the present invention include both unmodified PEG forms having a structure with general formula HO-(CH₂-CH₂-O)ₙ-H as indicated above. Additionally or alternatively one or more forms of PEG used in the present invention can comprise one or more PEG derivatives, more particularly PEG(s) substituted at one or more positions by one or more modifications which are alkyl substituents and/or functional substituents. A list of examples thereof comprises PEG 600 diacid, PEG 250 mono ethylether (MME) and PEG 350 dimethylether (DME).

For the use of the invention, the pharmaceutical compositions comprising PEG in a concentration of 0,5% to 5% (w/w), particularly between 1 and 3 % (w/w) are formulated in an aqueous solution, a hydrogel or hydrogel comprising composition. Such compositions are particularly suitable for topical administration. Compositions of suitable hydrogels for topical administration are known to the skilled person. As indicated above, hydrogels typically contain a network of natural or synthetic polymer chains dispersed in water. Hydrogels are generally used for retaining or absorbing moisture or water. Particularly suitable hydrogels in the context of the present invention are prepared with hydrocolloids such as alginates, carbomers (polyacrylic acids) (such as carbopol), cellulose and derivatives thereof such as carboxymethyl cellulose (CMC).

Other suitable hydrocolloids are alumina, betonite, starch, glycogen, gelatin, pectin, chitosan, chitin, gum Arabic, locust bean gum, karaya gum, gum tragacanth, ghatti gum, agar-agar, carrageenans, carob gum, guar gum, xanthan gum, glyceryl polymethacrylate, povidone, poloxamer (IUPAC:2-methyloxirane; oxirane); silicium dioxide/[Aluminium-Magnesium] silicates.

The viscosity of the hydrogel of the compositions envisaged for use in the present invention is appropriate for application on the skin. It has been found that the concentration of the hydrocolloid in the hydrogel also has an effect on the therapeutic wound healing activity. The viscosity of a hydrogel differs strongly depending on the type of colloid used. Particularly suitable hydrogels for use in the context of the present invention are hydrogels which have a viscosity between 4 and 4.000.000 mPa.s (water = 1 mPa.s). Typically, the envisaged viscosity is less than 450.000 mPa.s. A particular embodiment of the invention relates to the use of hydrogels having a viscosity of between 2000 - 300.000 mPa.s. Depending on the application and the nature of the hydrocolloid, hydrogels with a different viscocity can be used. For instance, in sprays, a hydrogel with a viscocity between 2000 - 6000, more particularly between 3000 and 5500 is used, while for gels for manual application based e.g. on a carbopol hydrocolloid, a hydrogel with a viscocity between 20.000 and 50.000 is typically used.

In particular embodiments, the hydrocolloids of the hydrogel used in the invention are at least partially cross-linked, e.g. with allyl ethers of pentaerythritol (carbopol^{®} 974P NF or Carbopol^{®} 980 NF). In particular embodiments the hydrocolloids are not cross-linked with each other or with a matrix (e.g. cellulose or cotton) in the hydrogel.

In particular embodiments the use of compositions is envisaged which are aqueous solutions comprising one or more forms of PEG, more particularly low Mr PEG in a concentration of 0,5-10% (w/w), particularly between 0,3 and 6%. Aqueous solutions envisaged are physiological solutions optionally comprising a buffer.

The hydrogel or aqueous composition comprising PEG as used in the present invention can be packaged as a tube, bottle or a disposable container. Alternatively it is envisaged that aqueous solutions or hydrogel compositions are provided on or within a gauze or textile or provided in a jar tube or in a spray container for spraying the hydrogel or aqueous solution on the skin.

In the Examples section herein, it is demonstrated that a composition comprising low Mr PEG, i.e. of Mr 1500 or less, more particularly a Mr of 700 or less, when present in low concentrations, i.e. typically below 5% (w/w) and particularly between 1 and 3 % (w/w)has an influence on the expression of certain proteins which are beneficial for the reduction of inflammation and in wound healing. Accordingly, the present invention provides for the use of PEG sole anti-inflammatory ingredient of a topical composition for the reduction or assistance in reduction of inflammation and/or to reduce or prevent scarring. More particularly, the invention provides for the use of PEG as sole anti-inflammatory ingredient in the manufacture of a topical medicament for treating inflammation and/or wound healing. Accordingly, compositions are provided comprising 0,5 - 10 % (w/w), particularly 0,3-6% (w/w), and more particularly between 1 and 3 (w/w) PEG, corresponding to one or more forms of PEG of between 200 and 700 MW, which are particularly suitable for the treatment of inflammatory skin and mucosal disorders.

According to particular embodiments, compositions used in the present invention are to be applied topically to skin or mucosa (including nose and ear), which are affected by inflammation. Inflammation of these tissues is characterized by the following quintet: redness (rubor), heat (calor), swelling (tumor), pain (dolor) and, where applicable, dysfunction of the organs involved (*functio laesa*).

Typical inflammatory skin disorders envisaged in the context of the present invention are eczema, acne, rosacea and many others including psoriasis, hives, contact dermatitis (such as occupational dermatitis) and poison ivy. Eczema, also known as atopic dermatitis, causes the skin to become dry, itchy and inflamed. Inflammatory diseases of the mucosa include inflammatory diseases of the nasal and paranasal sinuses, chronic inflammatory ear diseases such as chronic otitis media.

As detailed above, particular embodiments of the invention envisage the use of combinations of PEG and other active ingredients, such as sunscreens or warming agents. In particular embodiments, however the compositions used in the present invention do not comprise irritating agents such as those typically encountered in topical medicaments such as warming materials (e.g. capsaicin or menthol) and/or suncreens (e.g. benzoic acid based UV absorbing compounds, anthranilic acid based UV absorbing compounds, salicylic acid based UV absorbing compounds, cinnamic acid based UV absorbing compounds, benzophenone based UV absorbing compounds). Compositions used according to this aspect of the invention avoid the irritation of the skin during healing.

According to particular embodiments, the compositions used in the present invention are to be applied topically to a wound. Typical wounds envisaged in the present invention are both open and closed wounds, including chronic wounds like chronic leg ulcers, diabetic ulcers, pressure sores, acute wounds (such as grazes, knife cuts), that would benefit from wound healing promotion, wounds which are difficult to heal such as infected wounds, burn wounds (of different degrees) including sunburn, post-operative wounds, skin transplants and traumatological wounds.

The present invention uses compositions comprising 0,5-5 %, particularly 0,3-6% (w/w) PEG, and more particularly between 0,5 and 3 % (w/w), most particularly between 0,5 and 2% (w/w) and which are particularly suitable for use during the initial stage of wound healing when a reduction in matrix metalloproteinases (MMPs) can prevent the rupture of freshly generated matrix on wounds. The pharmaceutical compositions used in the present invention are also suitable for the treatment of scars, where the composition is applied at the later stages of wound healing.

As detailed above, the treatment of different types of inflammatory skin or mucosa disorders and wounds is envisaged for the compositions described herein. Wounds are typically characterized as either dry necrotic wounds, soft fibrinous (sloughy) wounds and granulating/epithelializing wounds. Depending on the nature of the wound bed, i.e. the amount of exudate formed, bacterial contamination etc. the frequency of application of the compositions described herein,can differ. Similarly, depending on the nature and severity of the inflammatory condition of the skin or mucosa, the frequency of application of the compositions described herein can differ.

The present invention envisages repeated application of the compositions disclosed herein where appropriate once every 5 days, most particularly between once every 24hrs and once every 72hrs. Alternatively, application is envisage once every 12hrs, or once every 4hrs, or once every 2hrs. Alternatively, the present compositions can be envisaged for one time use. Compositions as described herein can be packaged in tubes of 10g for single use or in tubes of e.g. 30-500g for multiple use, or in sprays for single or multiple use. Irrespective thereof, different types of packaging are envisaged including tubes, sprays (mouth, skin or ear), bottles, roll-on devices, sticks, suppositories or varginal devices, eardrops, eyedrops, etc..

Additionally or alternatively, compositions disclosed herein are applied to the area of the wound and/or the area of inflammation thereby avoiding or reducing the likelihood of scar formation.

Compositions disclosed herein are typically applied as wound-dressings, most particularly as hydrogels or aqueous solutions.

Hydrogels or hydrogel comprising compositions used in the present invention have the following advantages in the context of wound healing:
- they create a humid wound environment
- they do not stick aggressively to the wound, are easily removed
- they have a cleansing activity (by promoting autolytic debridement)
- they have a soothing effect by the cooling effect of the water in the hydrogel
- they have a moist-absorbing effect (by the swelling of the gel, which prevents the healthy skin borders from getting soft due to soaking).

Wounds heal better when they are kept humid. The skin cells that ensure the healing process grow quickly and scarring is reduced. In some skin wounds the skin is so dry that healing stagnates or stops. This can cause the wound to become larger and/or deeper. By adding moisture from the outside, the skin cells can regain their healing activity.

The compositions which are used in the present invention are optionally covered by a gauze or provided as an impregnated gauze. The presence of the hydrogels or aqueous solutions of the present invention reduces the frequency with which the gauze needs to be changed, and is user-friendly, thereby facilitating wound or skin care. The improved healing benefits skin and/or wound repair.

The compositions comprising PEG as described herein are also suitable for *in vitro* and *in vivo* in the handling of artificial skin preparations. In view of the effect of PEG on expression of VEGF, collagen and inflammatory cytokines, the compositions are useful in the production for the generation and/or maintenance of artificial skin *in vitro* and in the care of skin grafts *in vivo.*

The present invention is illustrated by the following examples.

### EXAMPLES

### Example 1: Toxicity of PEG on cultivated cells

Cells were grown in DMEM (3T3 fibroblasts and HaCat keratinocytes) or in RPM11640 (J774 macrophages). Both media were supplemented with heat inactivated 10% Fetal Calf Serum (Sigma), 2mM L-Glutamine (Sigma) and antibiotics (Sigma). Cells were seeded subconfluent in 96 well plates and contacted for about 16 hours with PEG test compounds which were dissolved in growth media (expressed as w/w end concentrations).
Cell survival was assayed by the conversion of soluble tetrazolium salt into an insoluble and pink colored formazan (absorption measured at 570nm). PEG were purchased from Sigma and are of European Pharmacopoeia or USP grade.

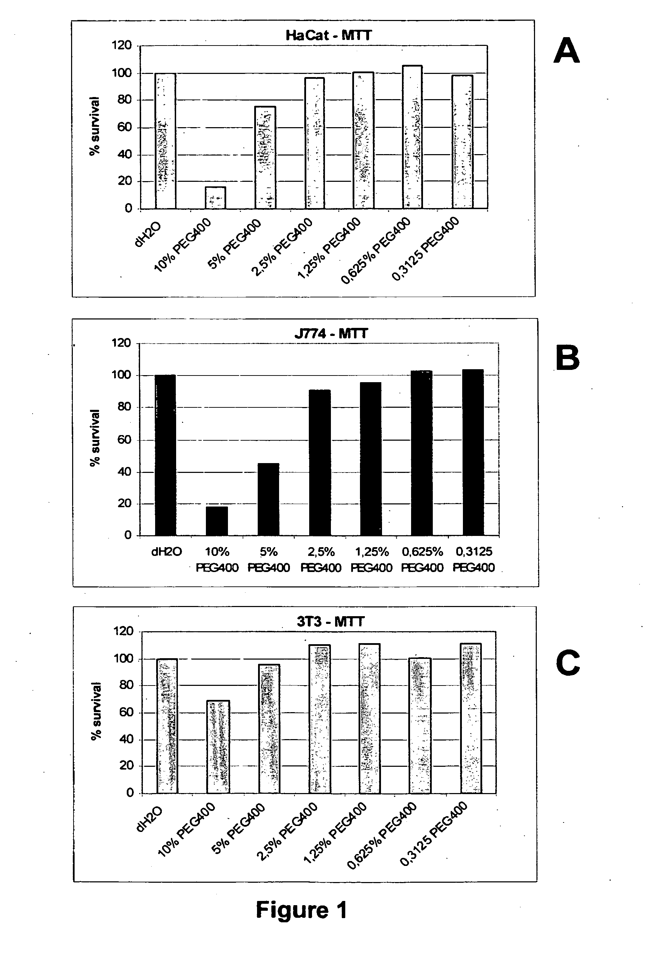

Cells were incubated overnight with varying concentrations of PEG 400 (0,31-10% w/w). From 5 % onwards, PEG has a cytotoxic effect on in vitro cultivated cells, especially on keratinocytes (figure 1A) and on macrophages (figure 1B), and to a lesser extent on fibroblasts (figure 1C). Concentrations below 5 % have no detrimental effect on any of the assayed cell types.

### Example 2: Influence of PEG on MMP expression

Macrophage cells were pre-conditioned for 30 min. with varying concentrations of PEG400 (0,31-10% w/w) and then challenged with heat inactivated E. coli bacteria. Next, cells were further incubated overnight. The supernatant of cells was analyzed for proMMP-9 according to the manufacturer's indications (R&D systems, Abingdon, UK).

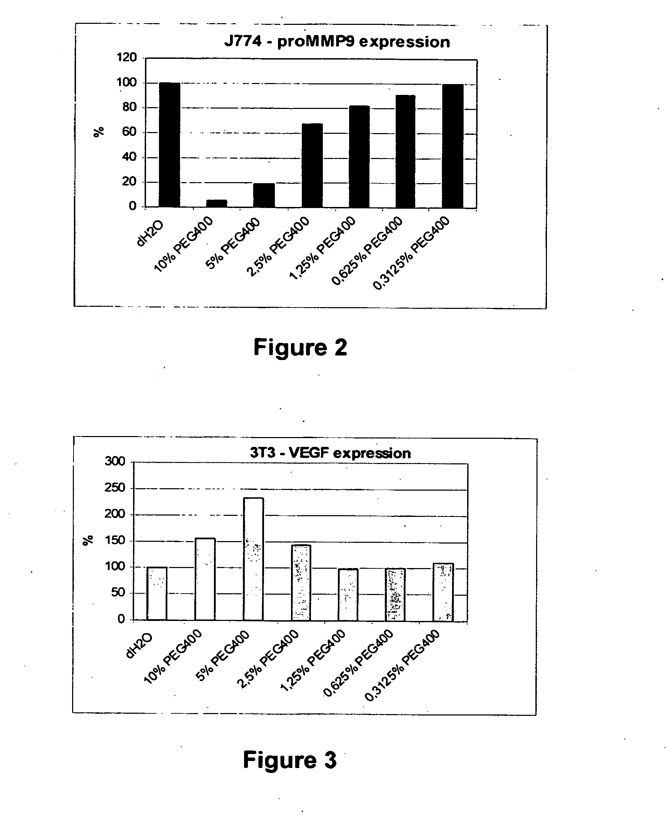

Figure 2 shows that secretion of proMMP9 by the J774 macrophage cell line was significantly reduced with PEG 400 concentration from about up to roughly 1% (w/w).
MMPs (matrix metalloproteinases) are extracellular proteinases that break down collagens. Although they are necessary during late stage wound healing, an increased amount of MMPs in chronic wounds or during the initial stages of wound healing is detrimental, since they rupture the freshly generated matrix. Accordingly, modulation of MMP expression is desired especially in chronic wound healing.

### Example 3: Influence of PEG on VEGF expression.

Fibroblasts cells were incubated overnight with varying concentration PEG 400 (0,31-10% w/w). The expression of VEGF was assayed by ELISA (R&D Sytems, Abingdon, UK).
Figure 3 shows that VEGF expression increases at PEG400 concentrations between 2 % and 10% with a maximum at 5 %.
VEGF is a cytokine with an important role during initial wound healing, by stimulating angiogenesis. Upon stimulation with VEGF, wounds make up granulation tissue more easily. Accordingly, upregulation of VEGF is desirable in wound healing.

### Example 4: Influence of PEG derivatives on cytokine expression.

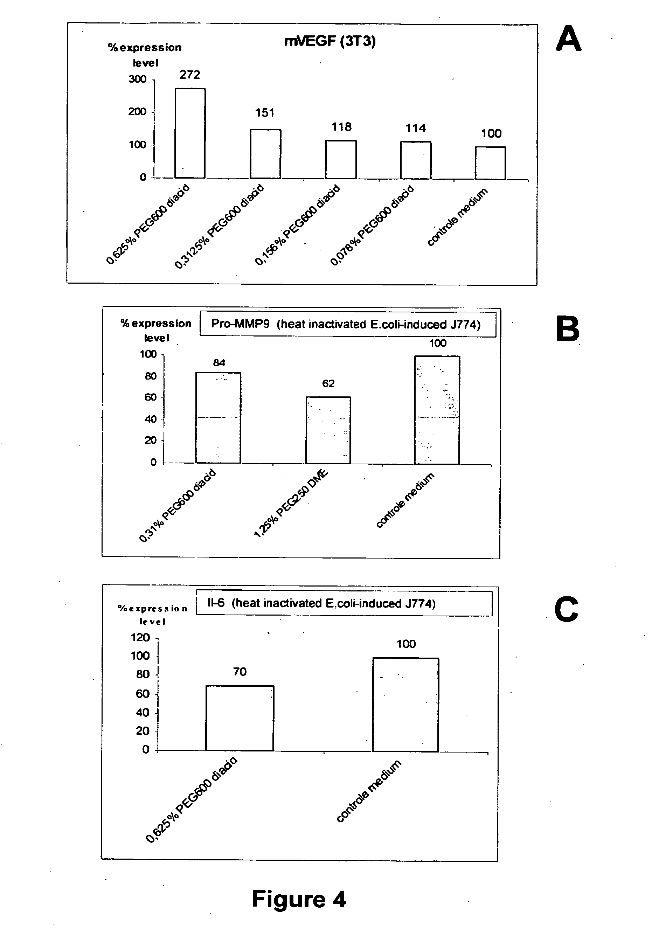

Cells were incubated overnight with varying concentrations of a PEG derivative Figure 4A shows that VEGF expression in non-inflammatory 3T3 fibroblast cells is influenced by increasing amounts of PEG 600 diacid (0,078-0,625% w/w).
Figure 4B shows that pro-MMP9 expression in J774 cells (challenged with heat inactivated *E*. *coli* bacteria after pre-incubation for 30 min.) is lowered upon incubation with PEG600 diacid or PEG 250 DME. As can be seen in the Figure, both PEG derivatives decrease the expression of the pro-inflammatory protein pro-MMP9.
Figure 4C shows the influence of PEG derivatives on the expression of the pro-inflammatory cytokine IL-6 by macrophage J774 cells after stimulation by a heat inactivated *E*. *coli* bacteria suspension. The cells were cultured with or without PEG (PEG600 diacid) The expression of IL-6 is lowered by about 30% when PEG is added.

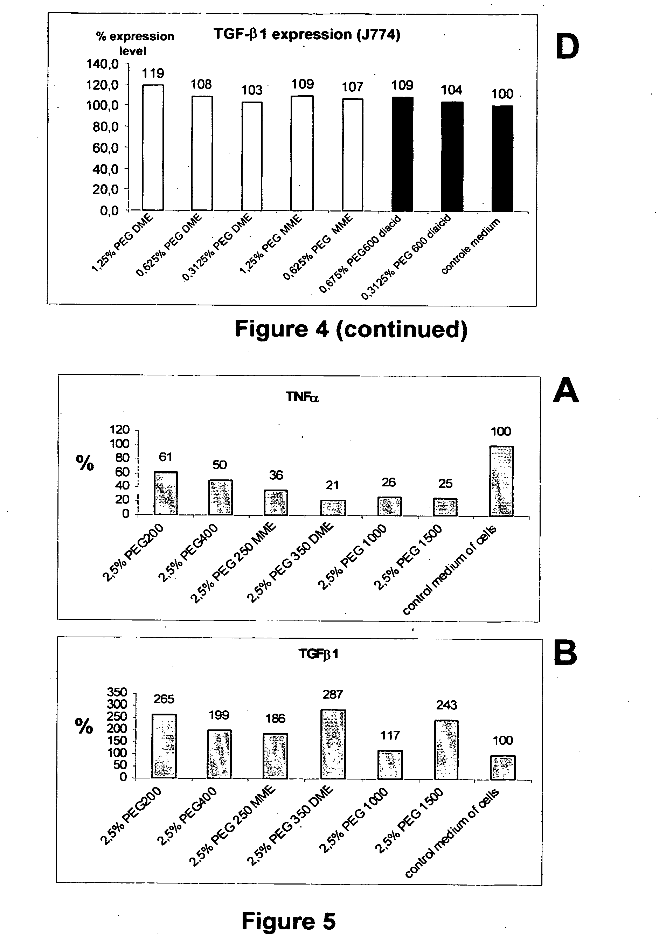

Figure 4d shows the influence of PEG derivatives (PEG 600 diacid, PEG350 MME and PEG250 DME) on the expression of the anti-inflammatory cytokine TGF-beta1.
PEG derivatives slightly increase the expression of TGF-beta 1 in macrophage J774 cells, in a concentration dependent manner. The effect is moderate, but provides evidence that PEG derivatives do not decrease expression of all cytokines in macrophages. The decreased expression of the pro-MMP9 seen in previous experiments can not be attributed to a general decrease in protein expression upon PEG addition.

### Example 5: Influence of PEG Molecular weight and PEG derivatives on human PBMCs (peripheral blood mononuclear cells).

PBMCs were isolated from buffy coats by Ficoll-Paque centrifugation according to the manufacturers protocol (Sigma). Hereafter, cells were washed twice with PBS/1mM EDTA to remove platelets. After overnight incubation at 37 °C, 5% CO₂, non-adherent cells were removed and cells were allowed to grow for an additional 21 days in growth medium (DMEM high glucose, 10% FCS, 4 mM L-glutamine, antibiotics). At this time point, cells are adherent to the cups of the wells and have a distinct morphology.
PEG and PEG derivatives were dissolved in growth medium at indicated concentrations (w/w). Cells were allowed to grow for 30 min in the PEG comprising medium (pre-conditioning) before 100 ng/ml LPS (Lipopolysaccharide) was added. Cells were incubated for an additional 16h. Afterwards, supernatant was collected and analyzed for indicated cytokines (R&D Systems, Abingdon, UK). Results are means of two independent experiments and are denoted as percentage compared to control.

The use of 2,5 % PEG of different types of PEG (Mr and derivatives) reduced pro-inflammatory TNF alpha secretion in PBMCs after stimulation with LPS (figure 5a). The anti-inflammatory cytokine TGF beta 1 was clearly upregulated by the PEGs and PEG derivatives.

### Example 6: Effect of PEG400 on protein expression in the presence of pharmaceutical excipients.

J774 macrophages were pre-conditioned for 30 min. with the gels and were then challenged with 100ng/ml LPS. 3T3 cells were not challenged with bacteria. Next, the murine macrophage J774 and murine fibroblast 3T3 cells were incubated with the gels for 16h at 37 °C, 5% CO₂. Hereafter, the medium was collected, centrifuged at 14.000 rpm during 20 min to remove particulate material and assayed for the indicated secreted proteins. Results are the mean of two independent experiments and are expressed as percentages of the control experiment.

### A: Na⁺-Ca⁺⁺ alginate gel

A hydrogel was made by dissolving 2 g Kelset (Na⁺-Ca⁺⁺ alginate) and 5 g PEG400 in a final volume of 100 ml growth medium [DMEM high glucose, 10% FCS, 4mM L-Glutamine, antibiotics]. This mixture was further diluted with an additional 100 ml of growth medium to lower the viscosity of the solutions. The final concentration of PEG400 was 2,5%.
A similar solution without PEG400 was used as control.

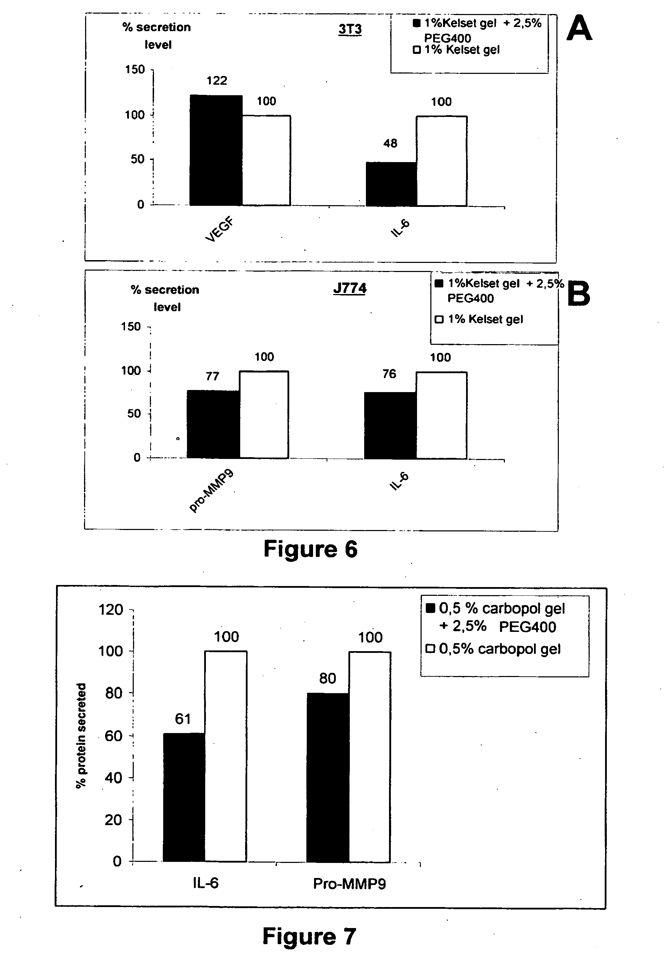

Figure 6A shows that the IL-6 expression in 3T3 cells was decreased, whereas the expression of the growth factor VEGF was slightly increased by the hydrogel that contained 2.5% PEG400.

Figure 6B shows that the secretion of both pro-inflammatory cytokines IL-6 and pro-MMP9 was reduced in J774 cells by PEG.

### B: Polyacrylate gel

A hydrogel was made by dissolving 1g Carbopol (polyacrylate), 5g PEG400, 2,1g Arginine and 3g PCL (cetearyl octanoate) in a total volume of 100 ml growth medium [DMEM(high glucose), 10% FCS, 4mM L-Glutamine, antibiotics]. This mixture was further diluted with an additional 100 ml of growth medium to lower the viscosity of the solutions. Cultures of murine macrophages J774 were used. The final concentration of PEG400 in contact with the cells was 2.5%. A similar solution without PEG400 was used as control.

Figure 7 shows that the expression of both pro-inflammatory cytokines IL-6 and pro-MMP9 was reduced after contacting the cells with a PEG containing hydrogel.

### C: Carboxymethylcellulose gel

A hydrogel was made by dissolving 3g CMC (Na⁺-carboxymethylcellulose), and 5g PEG400 in a final volume of 100 ml growth medium [DMEM(high glucose), 10% FCS/4mM L-Glutamine, antibiotics]. This mixture was further diluted with an additional 100 ml of growth medium to lower the viscosity of the solutions. Cultures of murine macrophages J774 were used. The final concentration of PEG400 was 2,5%. A similar solution without PEG400 was used as control.

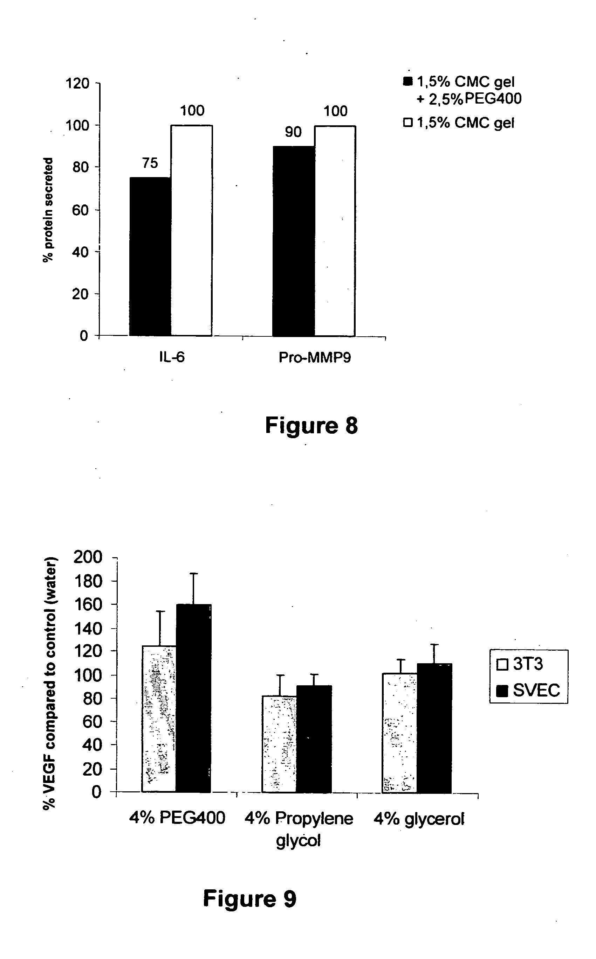

Figure 8 shows that the expression of the cytokines IL-6 was clearly reduced after cells had been challenged with 100ng/ml LPS, whereas the expression of pro-MMP9 was less reduced after contacting the cells with a PEG-containing hydrogel.

Comparison of the results of B and C shows that the "carrier" (carbopol versus CMC) also has an influence on proteins secreted (expressed) by J774 cells. Nevertheless, in every condition PEG400 decreases the expression of the pro-inflammatory proteins.

The above examples demonstrate that PEG400 modulates the expression of several proteins (in particular MMP's, inflammatory cytokines) which have a role in wound healing. Reducing matrix metalloproteinases levels in chronic wounds restores collagen deposition and reduces collagen destruction. This in turn promotes the generation of an optimal granulation bed. Increasing VEGF in the wound bed stimulates endothelial cells and enhances angiogenesis, resulting in increased blood flow towards the site of damage. Although PEG400 is cytotoxic from concentrations of about 5% (w/w) upwards, there is a therapeutic window (between about 0,3 and 6% PEG (w/w)) and particularly between 1 and 3 % (w/w) PEG400 wherein a beneficial effect of PEG400 is noticed.

### Example 7: Effect of PEG400 on VEGF and Collagen III protein expression. VEGF

The expression of VEGF upon administration of PEG and/or other humectants was investigated on vascular endothelial cells (SVEC) and on fibroblast cells (3T3). The beneficial role of VEGF expression in the healing of ischemic wounds is described in Corrall et al. (1999) Arch. Surg. 134, 200-205.

Administration of 4% PEG 400, 4% propylene glycol and 4% glycerol to these cells , shows that the highest expression of VEGF was obtained with PEG and that the difference in VEGF expression between endothelial cells and fibroblasts is most prominent with PEG400 (see Figure 9).

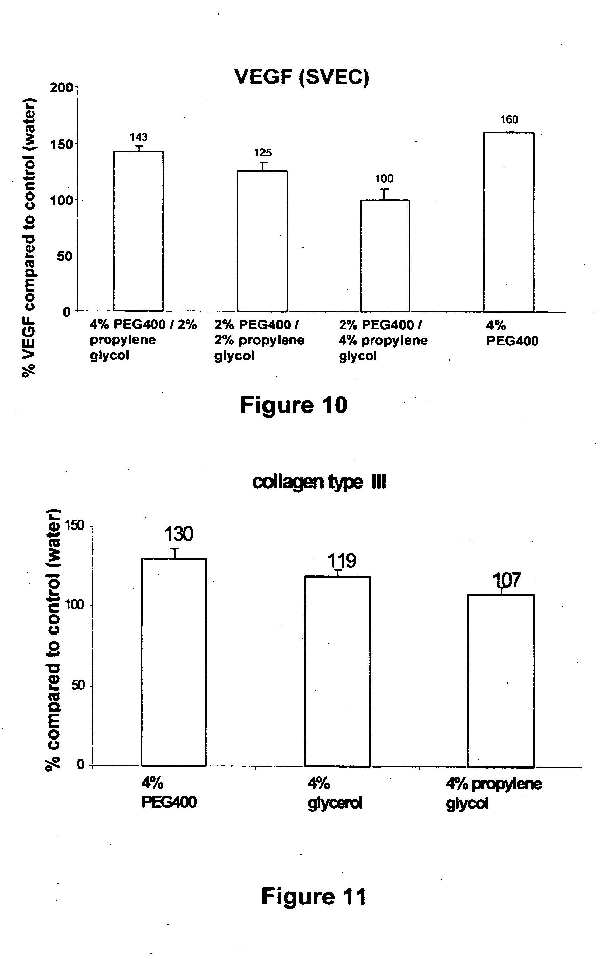

Figure 10 shows that combined formulations of PEG400 with propylene glycol have a lesser effect on VEGF expression than compositions comprising only PEG400.

### Collagen

Collagen type III expression is increased during the initial phase of wound healing in the granulation tissue (Haukiporo et al (1987) Ann. Surg. 206, 752-756). Chronic wounds are characterised by high collagenase activity and consequent collagen degradation. The use of collagen inducing compounds is consequently beneficial in both initial wound healing and treatment of chronic wounds. In addition, the increase in granulation tissue components is beneficial for a faster healing of the wound, which reduces the risk of complications resulting in scar formation.

Upon comparison of the administration of 4% PEG 400, 4% propylene glycol and 4% glycerol to 3T3 cells, it was found that the highest expression of collagen was obtained with PEG400 (Figure 11). This increase of collagen III expression was significant compared to control.

### Example 8: In vivo application of PEG compositions on wounds in a mouse wound healing model.

Wound healing of PEG comprising compositions is evaluated in a mouse model of wound healing. The assay is performed on wild type mice and on mutant mice with a mutation in the leptin receptor (BKS, Cg-m +/+ Leps) (Jackson Laboratory, Maine, USA). These mice are diabetic and have impaired wound healing.
Procedure: the hair on the back of the mice is shaved, the mice are anesthetized and full thickness wounds are applied with a skin punch biopsy apparatus. 6 wounds are applied per animal. After the application, a hydrogel composition is applied consisting of carbopol, alkalizing arginine and 1 % PEG. Mice are sacrificed on day 0, 7, 14 and 21 after application of the PEG or control composition on the wounds and the skin is evaluated visually and with immunohistochemical methods.

### Example 9: In vivo application of a PEG-comprising topical product on human skin.

A) A two year old boy suffered grazes on his leg after a fall on the street. A carbopol hydrogel containing 1% PEG400 was applied twice a day on the wound. No secondary dressing could be applied since the boy kept ripping it of. The wound healed nicely within 3 days without signs of swelling or any other inflammatory complication often encountered in such accidents.
B) A 28 year old professional soccer player suffered a relatively large graze through a sliding action on a dry football field. The wound was covered with slough and debris. After a brief rinsing, a carbopol hydrogel containing 1% PEG400 was applied on the wound and was covered by a secondary dressing. The gel was applied once a day. The player reported healing without inflammatory complications within 3 days, whereas from experience it took 4-6 days in previous interventions.

## Claims

1. Use of one or more forms of Polyethylene Glycol (PEG) with a Mr below 1500 as the sole anti-inflammatory component for the manufacture of a medicament in the form of a hydrogel or an aqueous solution comprising said one or more forms of Polyethylene Glycol (PEG) at a concentration between 0,5 and 5 % (w/w), for the treatment of inflammation of the skin or mucosa.

2. The use according to claim 1, comprising said one or more forms of Polyethylene Glycol (PEG) in said medicament at a concentration between 0,5 and 3% (w/w).

3. The use according to claim 1 or 2, comprising said one or more forms of Polyethylene Glycol (PEG) in said medicament at a concentration between 1 and 2,5 % (w/w).

4. The use according to any of claims 1 to 3, wherein said medicament contains no humectant apart from PEG.

5. The use according to any one of claims 1 to 4, wherein the one or more forms of PEG have a Mr between about 200 and 700.

6. The use according to any one of claims 1 to 5, wherein the hydrogel is a polyacrylate hydrocolloid with a concentration between 0,05-20%.

7. The use according to claim 1, wherein said inflammation occurs in a wound or inflammatory skin or mucosa disease.

8. The use according to claim 1, for the treatment of inflammation to prevent scar formation or to repair damaged skin or mucosa.

9. A hydrogel or aqueous solution comprising one or more forms of PEG with a Mr below 1500, in a concentration between 0,5 and 5% (w/w) as the sole anti-inflammatory component for use in reducing inflammation of the skin or mucosa.

10. The hydrogel or aqueous solution for use according to claim 9, wherein the concentration of said one or more forms of Polyethylene Glycol (PEG) is between 0,5 and 3 % (w/w).

11. The hydrogel or aqueous solution for use according to claims 9 or 10, wherein concentration of said one or more forms of Polyethylene Glycol (PEG) is between 1 and 2,5 % (w/w).

12. The hydrogel or aqueous solution for use according to any of claims 9 to 11, wherein the reduction in inflammation of the skin or mucosa promotes the healing of a wound and/or prevents scar tissue formation and/or enhances skin repair.

13. The hydrogel or aqueous solution according to any one of claims 9 to 11, for use reducing inflammation of the skin in an inflammatory skin disease.

14. The hydrogel or aqueous solution for use according to any one of claims 9 to 13, wherein said hydrogel or aqueous solution does not comprise a humectant apart from PEG.

15. The hydrogel or aqueous solution for use according to claim 12, wherein the wound is a chronic wound.

16. The hydrogel or aqueous solution for use according to any one of claims 9 to 15, which is a polyacrylate hydrocolloid with a concentration between 0,05 and 20%.

## Patentansprüche

1. Verwendung einer oder mehrerer Formen von Polyethylenglykol (PEG) mit einem Mr von weniger als 1500 als die einzige entzündungshemmende Komponente zur Herstellung eines Medikaments in Form eines Hydrogels oder einer wässrigen Lösung, aufweisend genannte eine oder mehrere Formen von Polyethylenglykol in einer Konzentration zwischen 0,5 und 5 % (w/w), zur Behandlung von Entzündungen der Haut oder der Schleimhaut.

2. Verwendung gemäß Anspruch 1, aufweisend genannte eine oder mehrere Formen von Polyethylenglykol (PEG) in genanntem Medikament in einer Konzentration zwischen 0,5 und 3 % (w/w).

3. Verwendung gemäß Anspruch 1 oder 2, aufweisend eine oder mehrere Formen von Polyethylenglykol (PEG) in genanntem Medikament in einer Konzentration zwischen 1 und 2,5 % (w/w).

4. Verwendung gemäß einem der Ansprüche 1 bis 3, wobei das genannte Medikament kein Feuchthaltemittel abgesehen von PEG enthält.

5. Verwendung gemäß einem der Ansprüche 1 bis 4, wobei die eine oder mehrere Formen von PEG einen Mr zwischen etwa 200 und 700 haben.

6. Verwendung gemäß einem der Ansprüche 1 bis 5, wobei das Hydrogel ein Polyacrylat-Hydrokolloid mit einer Konzentration zwischen 0,05 und 20 % ist.

7. Verwendung gemäß Anspruch 1, wobei die genannte Entzündung in einer Wunde oder einer entzündlichen Haut- oder Schleimhauterkrankung vorliegt.

8. Verwendung gemäß Anspruch 1 für die Behandlung einer Entzündung, um die Bildung von Narben zu vermeiden oder um beschädigte Haut oder Schleimhaut zu reparieren.

9. Ein Hydrogel oder eine wässrige Lösung, aufweisend eine oder mehrere Formen von PEG mit einem Mr von weniger als 1500 in einer Konzentration zwischen 0,5 und 5 % (w/w) als die einzige entzündungshemmende Komponente zur Verwendung bei der Reduktion von Entzündungen der Haut oder Schleimhaut.

10. Hydrogel oder wässrige Lösung zur Verwendung nach Anspruch 9, wobei die Konzentration genannter einer oder mehrerer Formen von Polyethylenglykol (PEG) zwischen 0.5 und 3 % (w/w) liegt.

11. Hydrogel oder wässrige Lösung zur Verwendung nach einem der Ansprüche 9 oder 10, wobei die Konzentration genannter einer oder mehrerer Formen von Polyethylenglykol (PEG) zwischen 1 und 2.5 % (w/w) liegt.

12. Hydrogel oder wässrige Lösung zur Verwendung nach einem der Ansprüche 9 bis 11, wobei die Reduktion der Entzündungen der Haut oder Schleimhaut das Ausheilen einer Wunde fördert und/oder die Bildung des Narbengewebes verhindert und/oder die Reparatur der Haut verbessert.

13. Hydrogel oder wässrige Lösung gemäß einem der Ansprüche 9 bis 11, zur Verwendung bei der Reduktion von Entzündungen der Haut bei einer entzündlichen Hautkrankheit.

14. Hydrogel oder wässrige Lösung zur Verwendung nach einem der Ansprüche 9 bis 13, wobei genanntes Hydrogel oder genannte wässrige Lösung kein Feuchthaltemittel abgesehen von PEG enthält.

15. Hydrogel oder wässrige Lösung zur Verwendung nach Anspruch 12, wobei die Wunde eine chronische Wunde ist.

16. Hydrogel oder wässrige Lösung zur Verwendung nach einem der Ansprüche 9 bis 15, wobei das Hydrogel oder die Lösung ein Polyacrylat-Hydrokolloid mit einer Konzentration zwischen 0,05 und 20 % ist.

## Revendications

1. L'utilisation d'une ou plusieurs formes de Polyéthylène Glycol (PEG) ayant un Mr en dessous de 1500 comme seul composant anti-inflammatoire pour la fabrication d'un médicament sous la forme d'un hydrogel ou d'une solution aqueuse comprenant lesdites une ou plusieurs formes du Polyéthylène Glycol (PEG) à une concentration entre 0,5 et 5% (en poids), pour le traitement d'une inflammation de la peau ou d'une muqueuse.

2. L'utilisation selon la revendication 1, comprenant lesdites une ou plusieurs formes de Polyéthylène Glycol (PEG) dans ledit médicament à une concentration entre 0,5 et 3% (en poids).

3. L'utilisation selon la revendication 1 ou 2, comprenant lesdites une ou plusieurs formes du Polyéthylène Glycol (PEG) dans ledit médicament à une concentration entre 1 et 2,5% (en poids).

4. L'utilisation selon l'une quelconque des revendications 1 à 3, dans laquelle ledit médicament ne contient pas d'humectant en dehors du PEG.

5. L'utilisation selon l'une quelconque des revendications 1 à 4, dans laquelle lesdites une ou plusieurs formes du PEG ont un Mr entre environ 200 et 700.

6. L'utilisation selon l'une quelconque des revendications 1 à 5, dans laquelle l'hydrogel est un polyacrylate hydro-colloïde avec une concentration entre 0,05 et 20%

7. L'utilisation selon la revendication 1, dans laquelle l'inflammation se produit dans une plaie ou une inflammation de la peau ou une maladie d'une muqueuse.

8. L'utilisation selon la revendication 1, pour le traitement d'une inflammation pour prévenir la formation d'une cicatrice ou pour réparer la peau ou une muqueuse endommagées.

9. Un hydrogel ou une solution aqueuse comprenant une ou plusieurs formes de PEG avec un Mr en dessous de 1500, dans une concentration entre 0,5 et 5% (en poids) comme seul composant anti-inflammatoire pour être utilisé dans la réduction de l'inflammation de la peau ou d'une muqueuse.

10. L'hydrogel ou la solution aqueuse pour une utilisation selon la revendication 9, dans lequel la concentration desdites une ou plusieurs formes du Polyéthylène Glycol (PEG) est comprise entre 0,5 et 3% (en poids).

11. L'hydrogel ou la solution aqueuse pour une utilisation selon les revendications 9 ou 10, dans lequel la concentration desdites une ou plusieurs formes du Polyéthylène Glycol (PEG) est comprise entre 1 et 2,5 % (en poids).

12. L'hydrogel ou la solution aqueuse pour une utilisation selon l'une quelconque des revendications 9 à 11, dans lequel la réduction de l'inflammation de la peau ou de la muqueuse favorise la guérison d'une plaie et/ou prévient la formation de tissu cicatriciel et/ou améliore la réparation de la peau .

13. L'hydrogel ou la solution aqueuse selon l'une quelconque des revendications 9 à 11, pour être utilisé dans la réduction de l'inflammation de la peau dans une maladie inflammatoire de la peau.

14. L'hydrogel ou la solution aqueuse pour une utilisation selon l'une quelconque des revendications 9 à 13, dans laquelle l'hydrogel ou la solution aqueuse en question ne comprend pas d' humectant en dehors du PEG.

15. L'hydrogel ou la solution aqueuse pour une utilisation selon la revendication 12, dans laquelle la plaie est une plaie chronique.

16. L'hydrogel ou la solution aqueuse pour une utilisation selon l'une quelconque des revendications 9 à 15, qui est un hydro-colloïde de polyacrylate avec une concentration entre 0,05 et 20% .
